# FASCICULE DE BREVET EUROPEEN

(11) **EP 2 838 889 B1**
(45) Date de publication et mention de la délivrance du brevet: **03.05.2017**
(21) Numéro de dépôt: 13722500.9
(22) Date de dépôt: 18.04.2013
(51) Int. Cl.: C07D 307/68, A61K 31/341, A61Q 19/02

(54) **ACIDE LICHESTÉRINIQUE ET SES DÉRIVÉS COMME INHIBITEURS DE PIGMENTATION**
LICHESTERINSÄURE UND DERIVATE DAVON ALS PIGMENTIERUNGSINHIBITOREN
LICHESTERINIC ACID AND THE DERIVATIVES OF SAME AS PIGMENTATION INHIBITORS

(30) Priorité: 18.04.2012 FR 1253585
(43) Date de publication de la demande: 25.02.2015
(73) Titulaire: Centre National de la Recherche Scientifique (C.N.R.S.), 75794 Paris Cedex 16 (FR); Université de Rennes 1, 35000 Rennes (FR); Centre Hospitalier Universitaire Pontchaillou, 35000 Rennes (FR)
(72) Inventeur: BOUSTIE, Joël, F-35760 Montgermont (FR); GALIBERT-ANNE, Marie-Dominique, F-35700 Rennes (FR); LOHEZIC-LE DEVEHAT, Françoise, F-35230 Orgeres (FR); CHOLLET-KRUGLER, Marylène, F-35630 La Chapelle Chaussee (FR); TOMASI, Sophie, F-35000 Rennes (FR); MOUCHET, Nicolas, F-35500 Vitre (FR); LEGOUIN-GARDADENNEC, Béatrice, F-35380 Treffendel (FR)
(74) Mandataire: Cabinet Plasseraud
(86) Numéro de dépôt international: PCT/FR2013/050856
(87) Numéro de publication internationale: WO 2013/156738

(56) Documents cités:
- FR-A1- 2 957 078
- US-A1- 2011 212 194
- Abrahams A et al.: "Lichen-derived compounds, lichesterinic and photolichesterinic acids, mediate pigmentation", , 29 juillet 2008 (2008-07-29), XP002680662, Extrait de l'Internet: URL:http://www.aferp.univ-rennes1.fr/aferp nouveau/athens/poster/PB170-Rennes%20(2).p df [extrait le 2012-07-25]
- DATABASE WPI Week 199315 Thomson Scientific, London, GB; AN 1993-121254 XP002680663, KATAGIRI T. ET AL.: "Skin external agent contg. lichesteric acid has high melanism-inhibiting effect enabling treatment of pigmentation and whitening of skin", & JP 5 058872 A (POLA CHEM IND INC) 9 mars 1993 (1993-03-09)

## Description

L'invention se rapporte à un composé de formule (A) ou l'un de ses sels pour dépigmenter la peau et/ou les phanères.

La couleur de la peau humaine et de ses phanères (cheveux, ongles, poils...) est fonction du phototype de l'individu. Une classification simple fondée sur l'intensité de la pigmentation constitutive (couleur de la peau, des cheveux et des yeux) associée à la sensibilité au soleil et à la capacité à bronzer des individus a été proposée en 1975 par le Dr. Fitzpatrick. L'intensité de la pigmentation constitutive est modulée par différents facteurs dont les saisons de l'année et le degré d'ensoleillement, le sexe et l'âge. Elle est principalement déterminée par la concentration de mélanine produite par les mélanocytes. Les mélanocytes sont les cellules spécialisées qui, par l'intermédiaire d'organelles particuliers, les mélanosomes, synthétisent la mélanine.

L'hyperpigmentation de la peau est un trouble fréquent qui se manifeste par l'apparition de taches brunes sur la peau. Elle est due à une accumulation non homogène de mélanine dans la peau. Les taches pigmentées peuvent apparaître sur n'importe quelle partie du corps, en particulier sur le dos des mains, le visage, le décolleté et les têtes chauves.

Plusieurs facteurs peuvent contribuer au développement de lésions hyperpigmentées ; les plus fréquents sont la prédisposition génétique (les peaux asiatiques sont plus sujettes aux taches hyperpigmentaires que les peaux caucasiennes), les hormones (masque de grossesse par exemple), l'exposition au soleil (qui entraîne un photovieillissement) et le vieillissement cutané. La production de mélanine peut également être augmentée par un processus inflammatoire cutané, par exemple après un traumatisme, des éruptions inflammatoires ou d'autres phénomènes, tels que des irritations cutanées.

Une forme commune d'hyperpigmentation se compose de taches de vieillesse ou taches solaires (lentigo solaire). Elles sont dues aux dommages causés par le soleil et apparaissent généralement sur le dos des mains et des bras, sur le décolleté ou sur le visage. Ces taches sont plus foncées que les taches de rousseur ou éphélides, et persistent dans l'hiver.

Il existe donc un réel besoin pour un traitement efficace et sans risque des taches hyperpigmentaires.

A l'inverse, il existe des maladies hypopigmentaires, qui se caractérisent par des macules blanches localisées. Le vitiligo est la plus fréquente des hypomélanoses. La démarche thérapeutique a alors pour objectif de diminuer le contraste inesthétique de la peau atteinte par rapport à la peau normalement pigmentée, soit en repigmentant la peau atteinte, soit en dépigmentant la peau non atteinte. Le choix de la stratégie thérapeutique dépendra de la localisation et de l'étendue des zones dépigmentées. Ainsi, lorsque le vitiligo est extrêmement étendu, il devient illusoire d'essayer de le repigmenter, et il est donc judicieux d'uniformiser la couleur de la peau en dépigmentant la peau saine résiduelle. Les résultats actuels sont encourageants mais insuffisants ; il existe donc un réel besoin d'identifier des molécules bioactives.

Enfin, en Afrique, en particulier dans les pays du Maghreb, les pays du Moyen-Orient, aux Antilles, sur le continent américain (Etats-Unis d'Amérique, Amérique du sud, Amérique centrale), en Asie (Inde, Philippines), et en Europe parmi les populations originaires de ces pays, un éclaircissement de la teinte naturelle de la peau est souvent perçu comme un signe de réussite sociale, de respect et de meilleures conditions de vie. Cette pression sociétale conduit à l'utilisation de cosmétiques dépigmentants pour l'obtention d'une teinte de peau plus claire. Cependant, et malgré le nombre de produits disponibles présentant des propriétés dépigmentantes, peu sont utilisables en cosmétologie en raison de leur toxicité. Il existe donc un réel besoin d'identification de molécules bioactives utilisables en toute sécurité.

A cet égard, de nombreux actifs chimiques ont été proposés, tels que l'hydroquinone, l'acide rétinoïque ou l'acide azélaïque. D'excellents résultats sont certes obtenus par les solutions proposées dans l'art antérieur, mais il n'en demeure pas moins que la découverte de substances ayant un effet sur la pigmentation de la peau ou de ses phanères reste un objectif majeur de la recherche dans ce domaine.
En effet, les produits de l'art antérieur doivent souvent être utilisés avec précaution en raison d'effets toxiques possibles. Ils sont par ailleurs de plus en plus surveillés, voire interdits dans certains pays, comme par exemple l'hydroquinone, désormais proscrite de la formulation des produits cosmétiques au sein de l'Union européenne depuis le règlement (CE) n°1223/2009 du 30 novembre 2009.

De manière surprenante, les inventeurs ont maintenant découvert que certains composés dérivés de lichen inhibent la pigmentation de la peau ou de ses phanères.

La demande FR 2 957 078, déposée par les demandeurs, décrit l'utilisation d'acides paraconiques, et notamment d'un mélange d'acide protolichestérinique ou ses sels, diastéréoisomères ou dérivés, et d'acide lichestérinique ou ses sels, énantiomères ou dérivés, dans un ratio spécifique, comme activateurs de la pigmentation.
Parmi les dérivés figurent des composés de formule chimique définie.
Or, il apparaît que certains de ces composés, utilisés seuls *in vitro,* à une concentration inférieure à la dose de 5 µM initialement évaluée et de surcroît sous une forme optique spécifique, ont, de manière surprenante, une activité dépigmentante.

Ainsi, l'invention a pour objet l'utilisation cosmétique d'un composé choisi parmi les composés de formule suivante (A) et leurs sels, sous leur forme dextrogyre (+): 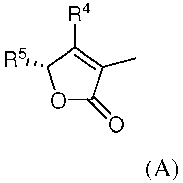
dans laquelle R⁴ représente un atome d'hydrogène, un radical *n*-butyle, un radical phényle non substitué, un radical phénéthyle non substitué ou un groupe COOR, où R = Na, H, méthyle ou éthyle,
et R⁵ est choisi parmi un atome d'hydrogène, les radicaux méthyle, éthyle, isopropyle, n-propyle, i-butyle,t-butyle, le radical -C₅H₁₁, le radical -C₉H₁₉, CH₂CCH, Ph, PhCH₂, PhCH₂CH₂, un radical (alkyle linéaire en C₃ à C₁₂)-CF₃, un radical alkyle linéaire en C₂ à C₁₃ avec une insaturation, en position terminale ou non, et (CH₂)₁₃COR⁶ où R⁶ = OCH₃, OH ou CH₃,
comme agent pour dépigmenter la peau et/ou les phanères.

L'invention a également pour objet un composé choisi parmi les composés de formule suivante (A) et leurs sels, sous leur forme dextrogyre (+): 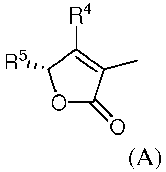
dans laquelle R⁴ représente un atome d'hydrogène, un radical *n*-butyle, un radical phényle non substitué, un radical phénéthyle non substitué ou un groupe COOR, où R = Na, H, méthyle ou éthyle,
et R⁵ est choisi parmi un atome d'hydrogène, les radicaux méthyle, éthyle, isopropyle, n-propyle, n-butyle, t-butyle, le radical -C₅H₁₁, le radical -C₉H₁₉, CH₂CCH, Ph, PhCH₂, PhCH₂CH₂, un radical (alkyle linéaire en C₃ à C₁₂)-CF₃, un radical alkyle linéaire en C₂ à C₁₃ avec une insaturation, en position terminale ou non, et (CH₂)₁₃COR⁶ où R⁶ = OCH₃, OH ou CH₃,
pour son utilisation pour dépigmenter la peau et/ou les phanères.

De préférence, le radical R⁵ avec une insaturation non terminale est le radical C₂H₅CHCHC₅H₁₀ en cis.
Par « phanères », on entend notamment les cheveux, les poils et les ongles.

Dans la présente demande, on entend par « acide lichestérinique » l'acide (+)-lichestérinique.
L'acide (+)-lichestérinique est en effet l'acide paraconique (i.e. possédant un cycle α-méthylène-γ-lactone β-substitué par une fonction acide) de formule (I) suivante :

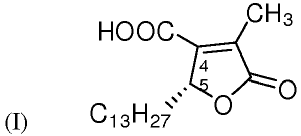

Ce composé est extrait de lichens, et notamment du lichen *Cetraria islandica* Ach. ou lichen d'Islande.

L'acide (+)-lichestérinique est de configuration *5R* grâce au carbone asymétrique en position 5 du cycle, la double liaison est de type endo.
Parmi les lichens producteurs d'acide (+)-lichestérinique, on trouve les lichens suivants : *Cetraria islandica, ericetorum, chlorophylla, cucullata, sepincola, ciliaris, laureri, nigricans, orbata, australiensis ou aculeata ; Cetraria komarovii (ou Nephromopsis komarovii); Parmelia camtschadalis et Nephromopsis stracheyi (ou Cetraria stracheyi f. ectocarpisma).*
L'acide (+)-lichestérinique peut être extrait à partir desdits lichens, notamment grâce au protocole décrit dans Horhant, D.; Le Lamer, A.-C.; Boustie, J. ; Uriac, P.; Gouault, N. « Séparation of a mixture of paraconic acids from Cetraria islandica (L.) Ach. employing a fluorous tag catch and release strategy. » Tetrahedron Lett. 2007, 48, 6031-6033.
Par « sels » des composés de formule (A), et notamment de l'acide lichestérinique, on entend les sels de ces composés avec des métaux alcalins tels que le sodium, le potassium ou le lithium, mais aussi les sels de ces composés avec des ions ammonium.

Dans la présente invention, sauf mention contraire, par « radical alkyle », on entend le radical méthyle, éthyle, isopropyle, n-propyle, n-butyle, i-butyle, t-butyle, le radical -C₅H₁₁ (pentyle), le radical -C₉H₁₉ (nonyle). De préférence, le radical alkyle est choisi parmi le radical n-butyle, le radical -C₅H₁₁, le radical -C₉H₁₉. Dans la présente invention, sauf mention contraire, par « radical alkyle en C₁ à C₅ linéaire ou ramifié », on entend le radical méthyle, éthyle, isopropyle, n-propyle, n-butyle, i-butyle, t-butyle ou le radical -C₅H₁₁ (pentyle).
Dans la présente invention, sauf mention contraire, par « radical (alkyle linéaire en C₃ à C₁₂)-CF₃ », on entend un radical alkyle linéaire ayant de 3 à 12 atomes de carbone, l'atome de carbone terminal étant substitué par un groupement CF₃.

Dans la présente invention, sauf mention contraire, par «radical alkyle linéaire en C₂ à C₁₃ avec une insaturation, en position terminale ou non », on entend un radical alkyle linéaire ayant de 2 à 13 atomes de carbone, possédant une insaturation en position terminale. En position non terminale, il s'agit, de préférence, du radical C₂H₅CHCHC₅H₁₀ en cis.

De préférence, le composé de formule (A) est tel que R⁴ est le groupe COOH, et R⁵ est un radical alkyle en C₁ à C₁₃ linéaire ou ramifié.

De préférence, le composé de formule (A) est choisi parmi l'acide (+)-3-methyl-5-nonyl-paraconique et l'acide (+)-3-methyl-5-pentyl-paraconique.

Le composé selon l'invention est utilisé pour inhiber la pigmentation de la peau et/ou des phanères. De préférence, le composé selon l'invention est utilisé pour traiter les désordres hyperpigmentaires.
Par désordres hyperpigmentaires, on entend de préférence le masque de grossesse, le lentigo solaire, le photovieillissement et les taches dues à une inflammation cutanée, une brûlure ou une irritation cutanée.
De préférence, le composé selon l'invention est également utilisé pour traiter les hypomélanoses telles que le vitiligo, où la peau saine sera alors la cible.
Enfin, le composé selon l'invention peut convenir pour une utilisation cosmétique visant à obtenir un blanchiment global de la peau pigmentée, pour répondre à une pression cosmétique sociétale.

Le composé selon l'invention peut être appliqué sur la peau ou les phanères par le biais d'une composition. Ladite composition comprend de préférence quantité comprise entre 0,0001% et 10% en poids par rapport au poids total de la composition, de préférence comprise entre 0.5 et 5% en poids, de composé selon l'invention.
Le composé selon l'invention peut être administré par le biais d'une composition par voie orale, par voie systémique ou par voie topique par application sur la peau et/ou les phanères.

Selon le mode d'administration, la composition comprenant le composé selon l'invention peut se présenter sous toutes les formes galéniques.
De préférence, le composé selon l'invention est compris dans une composition qui est administrée par voie topique sur la peau et/ou les phanères.

Par voie orale, les compositions peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée.

Pour une application topique sur la peau, la composition peut avoir la forme notamment de solution aqueuse ou huileuse ou de dispersion du type lotion ou sérum ; d'émulsion de consistance liquide ou semi-liquide du type lait, obtenue par dispersion d'une phase grasse dans une phase aqueuse (H/E) ou inversement (E/H) ; d'émulsion de consistance molle du type crème ; d'émulsion biphasée; de gel aqueux ou anhydre ; de mousse ou encore de microcapsules ou microparticules, ou de dispersions vésiculaires de type ionique et/ou non ionique, ou encore de formules pulvérisables de type « spray ». Ces compositions sont préparées selon les méthodes usuelles connues de l'homme de l'art.

Pour une application topique sur les cheveux, la composition peut avoir la forme de solutions aqueuses, alcooliques ou hydroalcooliques ; de gels ; d'émulsions ; de mousses ; ou encore sous forme de compositions pour aérosol comprenant également un agent propulseur sous pression. La composition selon l'invention peut aussi être une composition de soin capillaire, et notamment un shampooing, une lotion traitante, une crème ou un gel coiffant, ou encore une lotion ou un gel antichute.

Pour une application par voie systémique, la composition peut se présenter sous forme de solution aqueuse ou huileuse ou sous forme de sérum.

La composition comprenant le composé selon l'invention peut comprendre notamment une phase grasse, un émulsionnant, un solvant, un propénétrant ou encore un gélifiant (lipophile ou hydrophile).

Lorsque la composition comprend une phase grasse, cette dernière comprend au moins une huile ou une cire.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone), les huiles fluorées (perfluoropolyéthers), les cires d'abeille, de carnauba ou de paraffine, ainsi que les alcools gras et les acides gras (acide stéarique).

La composition peut également comprendre au moins un émulsionnant. Cet émulsionnant peut être anionique, cationique, non ionique ou amphotère.

Comme solvants utilisables selon l'invention, on peut citer les alcools inférieurs, notamment l'éthanol et l'isopropanol, et les propylènes glycols.

Comme propénétrants utilisables selon l'invention, on peut citer les glycols, notamment le 1,2-propanediol (ou propylène glycol) et les polyéthylènes glycols.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, l'éthylcellulose, le polyéthylene.

Selon l'invention la composition peut associer le composé selon l'invention à d'autres agents actifs.
Parmi ces agents actifs, on peut citer à titre d'exemple:
- les agents dépigmentants comme les rétinoïdes, l'acide azélaïque, l'acide kojique;
- les agents améliorant l'activité sur la repousse et/ou sur le freinage de la chute des cheveux, et ayant déjà été décrits pour cette activité comme par exemple le minoxidil, l'aminexil, les esters d'acide nicotinique, dont notamment le nicotinate de tocophérol, le nicotinate de benzyle et les nicotinates d'alkyles en C1-C6 comme les nicotinates de méthyle ou d'hexyle;
- les agents anti-inflammatoires stéroïdiens, tels que l'hydrocortisone, le valérate de bétaméthasone ou le propionate de clobétasol;
- les agents antifongiques tels que le kétoconazole, le sulfure de sélénium, l'itraconazole ou le fluconazole; ou
- les agents antiprurigineux comme la thénaldine, la triméprazine ou la cyproheptadine.

La composition peut comprendre également des adjuvants classiques, tels que les conservateurs, les antioxydants, les parfums, les charges, les absorbeurs d'odeur et les matières colorantes.
Les quantités de ces différents adjuvants sont celles classiquement utilisées, et par exemple de 0,01 % à 20 % en poids par rapport au poids total de la composition.

Plusieurs procédés seront envisagés selon la nature des radicaux R⁴ et R⁵ des composés de formule (A). Le procédé ci-dessous décrit la synthèse énantiosélective des composés de formule (A) avec R⁴ = COOH :
Ce procédé est issu, pour les étapes a, b, c et f, d'une publication de S. Braukmüller et R. Brückner, Eur. J. Org. Chem. 2006, 2110-2118.

Il s'agit d'une synthèse énantiosélective en 7 étapes dont l'énantiocontrôle est imposé par les étapes **b** et **d.** La condensation de l'aldéhyde sur l'hydrogénomalonate de méthyle (étape **a**) conduit à un ester carboxylique *trans.* -insaturé. Une dihydroxylation asymétrique de Sharpless permet l'obtention d'une ^{∼}hydroxy- ^{∼}lactone énantiopure (étape **b**)**.** Après déshydratation (étape **c**) et une *trans*-addition sélective du groupe vinyle (étape **d**), le composé est converti en acide paraconique (étape **e**). L' - activation en présence de carbonate de méthyle magnésium suivie d'une méthylénation décarboxylative conduit au dérivé de l'acide (+) ou (-)-protolichestérinique (étape **f**).

Une isomérisation de la double liaison exo en endo conduit à des dérivés de formule A (étape g). 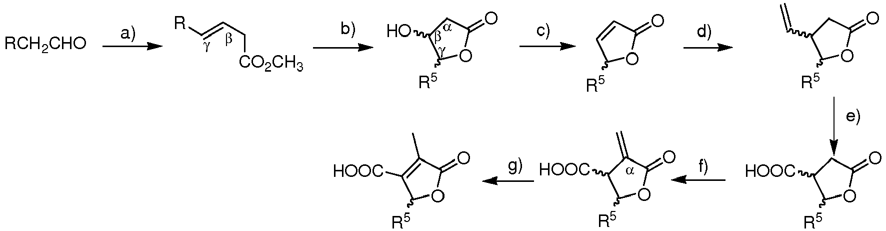 **a)** HOOCCH₂CO₂C₂H₅ (1.0 eq), NEt₃ (1.0 eq), 90°C, 3 h; **b)** AD mix- ® ou AD mix- ®, MeSO₂NH₂ (1.0 eq), *t*BuOH/H₂O (1:1), 0°C, 40 h ; **c)** MsCl (1.1 eq), NEt₃ (2.1 eq), CH₂Cl₂, 0 °C, 15 min; **d)** CuI (10 eq),THF, -78 °C, MeLi (10 eq), 15 min; bromure de vinyl magnésium (10 eq), 15 min, addition de furanone, 2 h; **e)** NaIO₄ (4.0 eq), RuCl₃ (0,1 eq), CCl₄/CH₃CN/H₂O (2:2 :3), TA 3 h; **f)** (i) MeOMg[O(C=O)Ome] (38 eq) dans le DMF, 135-140°C,70 h, isolement du brut (ii) brut, HOAc/NaOAc/formalin (= 35-40% Sol. aq. de formaldehyde)/N-methylaniline (excès; 4:0.03:3:1), TA, 2 h; **g)** NEt₃, DMF, 18 h.

Bien entendu, l'homme du métier veille à ne pas introduire des composés dans la composition utilisée dans la présente invention d'une manière telle que ces derniers contreviennent à l'effet technique désiré, objet de la présente invention.

On va maintenant donner à titre d'illustration des exemples qui ne sauraient limiter en aucune façon la portée de l'invention.

Les légendes des figures sont les suivantes :
**Figure 1** **: Taux de mélanine dans cellules B16 rapporté aux témoins (100%)** mesuré par absorbance après 4 jours d'incubation (Boîtes de Pétri) avec des concentrations inférieures ou égales à 2µM d'un mélange (+)AL/(+)APL/(+)AR en proportion respective 60/25/15.
**Figure 2** **: Taux de mélanine dans cellules B16 rapportés aux témoins (boîtes de Pétri)** mesuré par absorbance après 4 jours d'incubation à 3 concentrations d'AL+ et d'AL-, et à 2 concentrations de C9AL+ et C5AL+. Le témoin positif dépigmentant utilisé est l'hydroquinone.

Les abréviations sont les suivantes :
(+)AL ou AL+ = acide (+)-lichestérinique (ou acide lichestérinique dextrogyre)
(-)AL ou AL- = acide (-)-lichestérinique (ou acide lichestérinique lévogyre)
(+)APL ou APL+ = acide (+)-protolichestérinique (ou acide protolichestérinique dextrogyre)
(+)AR ou AR+ = acide (+)-roccellarique (ou acide roccellarique dextrogyre) C9AL+ = acide (+)-3-méthyl-5-nonyl-paraconique (ou acide 3-méthyl-5-nonyl-paraconique dextrogyre)
C5AL+ = acide (+)-3-méthyl-5-pentyl-paraconique (ou acide 3-méthyl-5-nonyl-paraconique dextrogyre)
et correspondent aux formules suivantes:

| | | |
|---|---|---|
| AL+ | AL- | APL+ |
| 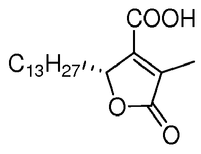 | 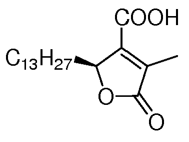 | 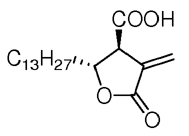 |
| | | |
| AR+ | C9AL+ | C5AL+ |
| 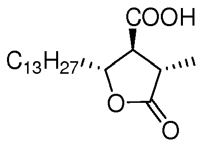 | 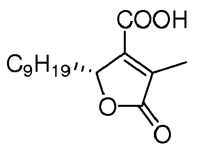 | 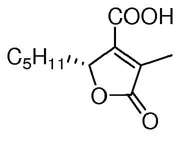 |

### EXEMPLE 1 : Mise en évidence de l'activité dépigmentante in vitro de l'acide (+)-lichestérinique ((+)AL)

Protocole : Le contenu en mélanine des cellules B16 murines est déterminé par spectrophotométrie, et toutes les concentrations retenues pour les essais ne présentent pas, dans les conditions décrites, de toxicité cellulaire (viabilité des B16 > 80%).
Les cellules sont ensemencées en boîte de Pétri de 10 cm à une densité de 7x 10⁵ par boîte et sont incubées pendant 96 heures, avec une concentration définie de molécule dérivée de lichen, purifiée et solubilisée dans du DMSO. Après traitement, les cellules sont récupérées par un traitement à la trypsine et lavées 2 fois avec du PBS. Le nombre de cellules récupérées est estimé par comptage à l'aide d'un hémocytomètre. Une même fraction est utilisée pour déterminer le contenu en mélanine et la concentration en protéine. Le contenu en mélanine est déterminé par l'absorbance à 405 nm (VersaMax Microplate Reader, Molecular Devices, USA) de la solution cellulaire après solubilisation de la mélanine par de la soude 1M, pendant 15min à 80°C. La concentration en protéine est déterminée selon le protocole du kit « DC Protein Assay » développé par les laboratoires Bio-Rad, USA. Pour chaque échantillon traité, le contenu en mélanine est rapporté à la quantité de protéine et exprimé en pourcentage par rapport à la situation contrôle (solution DMSO seule). Chaque mesure est réalisée en triplicata et chaque expérience est réalisée indépendamment un minimum de 3 fois (sauf pour les dérivés C9AL+ et C5AL+ donnés à titre indicatif).

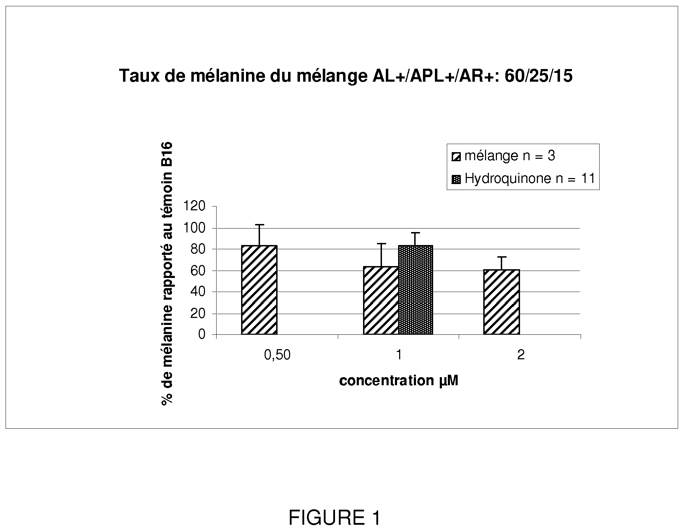

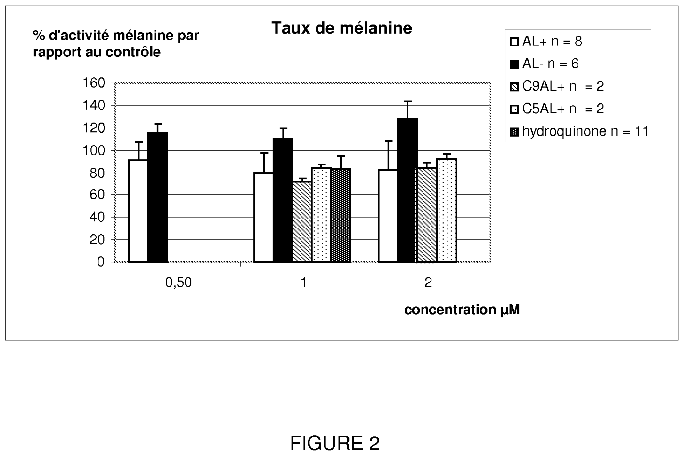

Les Figures 1 et 2 rassemblent les résultats. Ces figures montrent le contenu en mélanine des cellules B16 traitées pendant 96h en présence d'une molécule donnée (Figure 2), ou d'un mélange (+)-acide lichestérinique ((+)AL)/(+)-acide protolichestérinique ((+)APL)/(+)-acide roccellarique ((+)AR) dans un ratio pondéral 60/25/15 (Figure 1).
En abscisse figurent les molécules testées avec leurs concentrations exprimées en µM. En ordonnée, le contenu en mélanine est exprimé en pourcentage par rapport au témoin (DMSO seul = concentration de 0.1%).

Les résultats de la Figure 1 montrent que pour un mélange contenant de l'acide (+)-lichestérinique, de l'acide (+)-protolichestérinique et de l'acide (+)-roccellarique dans les proportions respectives 60/25/15, une inhibition de l'ordre de -20 à -50% du taux de mélanine est enregistrée aux concentrations de 1 et 2 µM.

Les résultats de la Figure 2 montrent quant à eux que les deux énantiomères de l'acide lichestérinique donnent des activités opposées, soit +10 à +30 % de stimulation de la production de mélanine pour l'acide (-)-lichestérinique (AL-) à des concentrations de 0.5 à 2µM, et 10% à 20% de baisse du taux de mélanine pour l'acide (+)-protolichestérinique (APL+) aux mêmes concentrations.
Par rapport au témoin positif dépigmentant (hydroquinone, induisant une diminution de -20% du taux de mélanine dans les mêmes conditions), l'acide (+)-lichestérinique, l'acide (+)-3-méthyl-5-nonyl-paraconique (C9AL+) et l'acide (+)-3-méthyl-5-pentyl-paraconique (C5AL+) présentent une activité dépigmentante comparable (cas de C5AL+) ou supérieure.

Dans le même temps, d'autres résultats non donnés ici indiquent que, pour les 2 autres produits constitutifs du mélange et isolés individuellement, les essais dans les mêmes conditions aboutissent à une baisse du taux de mélanine de l'ordre de -20% pour l'acide (+)-protolichestérinique à 0,25µM, et non significative pour l'acide roccellarique à 1µM, leurs énantiomères respectifs ayant eux aussi le même niveau d'activité à ces mêmes concentrations.

Les résultats obtenus démontrent donc l'activité dépigmentante de l'acide (+)-lichestérinique et de ses dérivés selon l'invention.

## Revendications

1. Utilisation cosmétique d'un composé choisi parmi les composés de formule suivante (A) et leurs sels, sous leur forme dextrogyre (+): 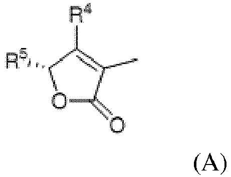
dans laquelle R⁴ représente un atome d'hydrogène, un radical *n*-butyle, un radical phényle non substitué, un radical phénéthyle non substitué ou un groupe COOR, où R = Na, H, méthyle ou éthyle,
et R⁵ est choisi parmi un atome d'hydrogène, les radicaux méthyle, éthyle, isopropyle, n-propyle, n-butyle, i-butyle, t-butyle, le radical -C₅H₁₁, le radical -C₉H₁₉, CH₂CCH, Ph, PhCH₂, PhCH₂CH₂, un radical (alkyle linéaire en C₃ à C₁₂)-CF₃, un radical linéaire alkyle en C₂ à C₁₃ avec une insaturation, en position terminale ou non, et (CH₂)₁₃COR⁶ où R⁶ = OCH₃, OH ou CH₃,
et les composés de formule (A) et leurs sels, sous leur forme lévogyre (-), dans laquelle R4 est tel que décrit précédemment et R5 est un radical alkyle en C₁ à C₅ linéaire ou ramifié, comme agent pour dépigmenter la peau et/ou les phanères.

2. Composé choisi parmi les composés de formule suivante (A) et leurs sels, sous leur forme dextrogyre (+): 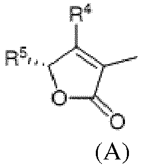
dans laquelle R⁴ représente un atome d'hydrogène, un radical *n*-butyle, un radical phényle non substitué, un radical phénéthyle non substitué ou un groupe COOR, où R = Na, H, méthyle ou éthyle,
et R⁵ est choisi parmi un atome d'hydrogène, les radicaux méthyle, éthyle, isopropyle, n-propyle, n-butyle, i-butyle, t-butyle, le radical -C₅H₁₁, le radical -C₉H₁₉, CH₂CCH, Ph, PhCH₂, PhCH₂CH₂, un radical (alkyle linéaire en C₃ à C₁₂)-CF₃, un radical alkyle linéaire en C₂ à C₁₃ avec une insaturation, en position terminale ou non, et (CH₂)₁₃COR⁶ où R⁶ = OCH₃, OH ou CH₃,
et les composés de formule (A) et leurs sels, sous leur forme lévogyre (-), dans laquelle R4 est tel que décrit précédemment et R5 est un radical alkyle en C₁ à C₅ linéaire ou ramifié, pour son utilisation pour dépigmenter la peau et/ou les phanères.

3. Utilisation cosmétique d'un composé selon la revendication 1 ou composé pour son utilisation selon la revendication 2, **caractérisé en ce que** R⁵ est un radical alkyle en C₂ à C₁₃ avec une insaturation non terminale en cis, de préférence le radical C₂H₅CHCHC₅H₁₀- en cis.

4. Utilisation cosmétique d'un composé selon la revendication 1 ou 3, ou composé pour son utilisation selon l'une des revendications 2 à 3, **caractérisé en ce que** le composé ou son sel, sous forme dextrogyre, est présent en une quantité comprise entre 0,0001% et 10% en poids par rapport au poids total de la composition.

5. Utilisation cosmétique d'un composé selon la revendication 1, ou composé pour son utilisation selon la revendication 2, **caractérisé en ce que** R⁴ est le groupe COOH, et R⁵ est choisi parmi les radicaux méthyle, éthyle, isopropyle, n-propyle, n-butyle, i-butyle, t-butyle, le radical -C₅H₁₁ et le radical -C₉H₁₉.

6. Utilisation cosmétique d'un composé selon la revendication 1 ou 5, ou composé pour son utilisation selon la revendication 2 ou 5, **caractérisé en ce que** le composé est choisi parmi l'acide (+)-3-methyl-5-nonyl-paraconique et l'acide (+)-3-methyl-5-pentyl-paraconique.

## Patentansprüche

1. Kosmetische Verwendung einer Verbindung, ausgewählt aus den Verbindungen der folgenden Formel (A) sowie ihren Salzen, in ihrer rechtsdrehenden Form (+): 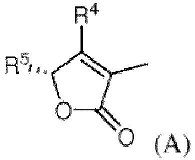
worin R⁴ für ein Wasserstoffatom, ein n-Butylradikal, ein unsubstituiertes Phenylradikal, ein unsubstituiertes Phenethylradikal oder eine COOR-Gruppe steht, wobei R = Na, H, Methyl oder Ethyl,
und R⁵ ausgewählt ist aus einem Wasserstoffatom, den Methyl-, Ethyl- Isopropyl-, n-Propyl-, n-Butyl-, i-Butyl-, und t-Butylradikalen, dem -C₅H₁₁-Radikal, dem -C₉H₁₉-Radikal, CH₂CCH, Ph, PhCH₂, PhCH₂CH₂, einem (linearen C₃-C₁₂-Alkyl) -CF₃-Radikal, einem linearen C₂-C₁₃ Alkylradikal mit oder ohne einer ungesättigten endständigen Gruppe, und (CH₂)₁₃COR⁶, wobei R⁶ = OCH₃, OH oder CH₃,
und den Verbindungen der Formel (A) sowie ihren Salzen, in ihrer linksdrehenden Form (-), worin R⁴ für oben beschriebenes steht, und R⁵ ein lineares oder verzweigtes C₁-C₅ Alkylradikal ist,
als Mittel zur Depigmentierung der Haut und/oder der Hautanhangsgebilde.

2. Verbindung, ausgewählt aus den Verbindungen der folgenden Formel (A) und ihrer Salze, in ihrer rechtsdrehenden Form (+): 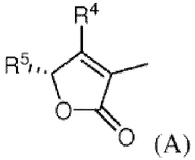
worin R⁴ für ein Wasserstoffatom, ein n-Butylradikal, ein unsubstituiertes Phenylradikal, ein unsubstituiertes Phenethylradikal oder eine COOR-Gruppe steht, wobei R = Na, H, Methyl oder Ethyl,
und R⁵ ausgewählt ist aus einem Wasserstoffatom, den Methyl-, Ethyl- Isopropyl-, n-Propyl-, n-Butyl-, i-Butyl-, und t-Butylradikalen, dem -C₅H₁₁-Radikal, dem -C₉H₁₉-Radikal, CH₂CCH, Ph, PhCH₂, PhCH₂CH₂, einem (linearen C₃-C₁₂-Alkyl) -CF₃ -Radikal, einem linearen C₂-C₁₃ Alkylradikal mit oder ohne einer ungesättigten endständigen Gruppe, und (CH₂)₁₃COR⁶, wobei R⁶ = OCH₃, OH oder CH₃,
und den Verbindungen der Formel (A) sowie ihren Salzen, in ihrer linksdrehenden Form (-), worin R⁴ für oben beschriebenes steht, und R⁵ ein lineares oder verzweigtes C₁-C₅ Alkylradikal ist,
zur Verwendung zur Depigmentierung der Haut und/oder der Hautanhangsgebilde.

3. Kosmetische Verwendung einer Verbindung gemäß Anspruch 1 oder Verbindung zur Verwendung gemäß Anspruch 2, dadurch charakterisiert, dass R⁵ ein nicht endständig cisungesättigtes C₂-C₁₃-Alkylradikal ist, vorzugsweise das cis-C₂H₅CHCHC₅H₁₀-Radikal.

4. Kosmetische Verwendung einer Verbindung gemäß Anspruch 1 oder 3, oder Verbindung zur Verwendung gemäß einem der Ansprüche 2 bis 3, dadurch charakterisiert, dass die Verbindung oder ihr Salz, in rechtsdrehender Form, in einer Menge zwischen 0,0001 Gew.-% und 10 Gew.-%, bezogen auf das Gesamtgewicht der Zusammensetzung, vorliegt.

5. Kosmetische Verwendung einer Verbindung gemäß Anspruch 1 oder Verbindung zur Verwendung gemäß Anspruch 2, dadurch charakterisiert, dass R⁴ die COOH-Gruppe ist, und R⁵ aus den Methyl-, Ethyl-, Isopropyl-, n-Propyl-, n-Butyl-, i-Butyl-, t-Butylradikalen, dem -C₅H₁₁-Radikal und dem -C₉H₁₉-Radikal ausgewählt ist.

6. Kosmetische Verwendung einer Verbindung gemäß Anspruch 1 oder 5, oder Verbindung zur Verwendung gemäß Anspruch 2 oder 5, dadurch charakterisiert, dass die Verbindung aus (+)-2-Oxo-2,5-dihydro-3-methyl-5-nonyl-4-furancarbonsäure ("l'acide (+)-3-methyl-5-nonyl-paraconique") und (+)-2-Oxo-2,5-dihydro-3-methyl-5-pentyl-4-furancarbonsäure ("l'acide (+)-3-methyl-5-pentyl-paraconique") ausgewählt ist.

## Claims

1. The cosmetic use of a compound chosen from the compounds of following formula (A) and salts thereof, in their dextrorotatory (+) form: 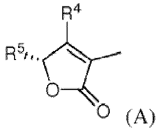
in which R⁴ represents a hydrogen atom, an n-butyl radical, an unsubstituted phenyl radical, an unsubstituted phenethyl radical or a COOR group, where R = Na, H, methyl or ethyl,
and R⁵ is chosen from a hydrogen atom, the methyl, ethyl, isopropyl, n-propyl, n-butyl, i-butyl and t-butyl radicals, the -C₅H₁₁ radical, the -C₉H₁₉ radical, CH₂CCH, Ph, PhCH₂, PhCH₂CH₂, a (linear C₃ to C₁₂ alkyl)-CF₃ radical, a linear C₂ to C₁₃ alkyl radical with an unsaturation, in the terminal or non-terminal position, and (CH₂)₁₃COR⁶ where R⁶ = OCH₃, OH or CH₃,
and the compounds of formula (A) and salts thereof, in their levorotatory (-) form, in which R⁴ is as previously described and R⁵ is a linear or branched C₁ to C₅ alkyl radical,
as an agent for depigmenting the skin and/or the appendages.

2. A compound chosen from the compounds of following formula (A) and salts thereof, in their dextrorotatory (+) form: 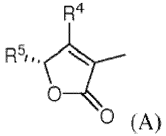
in which R⁴ represents a hydrogen atom, an *n*-butyl radical, an unsubstituted phenyl radical, an unsubstituted phenethyl radical or a COOR group, where R = Na, H, methyl or ethyl,
and R⁵ is chosen from a hydrogen atom, the methyl, ethyl, isopropyl, n-propyl, n-butyl, i-butyl and t-butyl radicals, the -C₅H₁₁ radical, the -C₉H₁₉ radical, CH₂CCH, Ph, PhCH₂, PhCH₂CH₂, a (linear C₃ to C₁₂ alkyl)-CF₃ radical, a linear C₂ to C₁₃ alkyl radical with an unsaturation, in the terminal or non-terminal position, and (CH₂)₁₃COR⁶ where R⁶ = OCH₃, OH or CH₃,
and the compounds of formula (A) and salts thereof, in their levorotatory (-) form, in which R⁴ is as previously described and R⁵ is a linear or branched C₁ to C₅ alkyl radical,
for use in depigmenting the skin and/or the appendages.

3. The cosmetic use of a compound as claimed in claim 1 or the compound for use as claimed in claim 2, **characterized in that** R⁵ is a C₂ to C₁₃ alkyl radical with a non-terminal cis-unsaturation, preferably the cis-C₂H₅CHCHC₅H₁₀- radical.

4. The cosmetic use of a compound as claimed in claim 1 or 3, or the compound for use as claimed in one of claims 2 to 3, **characterized in that** the compound or salt thereof, in dextrorotatory form, is present in an amount of between 0.0001% and 10% by weight relative to the total weight of the composition.

5. The cosmetic use of a compound as claimed in claim 1, or the compound for use as claimed in claim 2, **characterized in that** R⁴ is the COOH group, and R⁵ is chosen from the methyl, ethyl, isopropyl, n-propyl, n-butyl, i-butyl and t-butyl radicals, the -C₅H₁₁ radical and the -C₉H₁₉ radical.

6. The cosmetic use of a compound as claimed in claim 1 or 5, or the compound for use as claimed in claim 2 or 5, **characterized in that** the compound is chosen from (+)-3-methyl-5-nonylparaconic acid and (+)-3-methyl-5-pentylparaconic acid.
